# EUROPÄISCHE PATENTSCHRIFT

(11) **EP 3 096 809 B1**
(45) Veröffentlichungstag und Bekanntmachung des Hinweises auf die Patenterteilung: **06.03.2019**
(21) Anmeldenummer: 15700184.3
(22) Anmeldetag: 06.01.2015
(51) Int. Cl.: A61M 1/16, B01D 63/02, B01D 69/02, B01D 69/08

(54) **DIALYSEGERÄT MIT EINEM DIALYSATOR**
DIALYSIS APPARATUS COMPRISING A DIALYZER
APPAREIL DE DIALYSE MUNI D'UN DIALYSEUR

(30) Priorität: 21.01.2014 DE 102014000737
(43) Veröffentlichungstag der Anmeldung: 30.11.2016
(73) Patentinhaber: Nephro-Solutions AG, 20149 Hamburg (DE)
(72) Erfinder: GOTTSCHALK, Oliver, 22453 Hamburg (DE)
(74) Vertreter: Groth, Wieland
(86) Internationale Anmeldenummer: PCT/EP2015/050083
(87) Internationale Veröffentlichungsnummer: WO 2015/110277

(56) Entgegenhaltungen:
- EP-A1- 0 842 694
- EP-A1- 2 540 327
- WO-A1-2013/106109
- US-B1- 6 309 543
- US-B1- 6 454 998
- SUSANNE H. WOLFF ET AL: "Effects of Bleach Reprocessing on Hemodialysis Membranes", MRS PROCEEDINGS, Bd. 752, 1. Januar 2002 (2002-01-01), XP055181257, DOI: 10.1557/PROC-752-AA14.3

## Beschreibung

Die Erfindung betrifft ein Dialysegerät nach Anspruch 1 mit einem Dialysator mit einem Bündel Kapillarmembranen mit jeweils einer Außenfläche und einer Innenfläche und mit einer im Bereich der Außenfläche größeren durchschnittlichen Porengröße als im Bereich der Innenfläche eines Blutkreislaufes und eines Dialysatkreislaufes.

Dialysegeräte sind im Stand der Technik natürlich hinlänglich bekannt. Weltweit verdanken zurzeit ca. 2,5 Mio. Dialysepatienten einer Nierenersatztherapie ihr Leben. Zur Durchführung der Nierenersatztherapie werden zum größten Teil Dialysatoren mit Kapillarmembranen eingesetzt, die bei der Basisbehandlung durch sogenannte "Low-Flux-Dialysatoren" die niedermolekularen toxischen harnpflichtigen Substanzen, wie beispielsweise Harnstoff und Kreatinin, aus dem Blut entfernen.

Alternativ dazu lassen High-Flux-Membranen wegen ihrer größeren Porengrößen in der Membranstruktur zusätzlich einen Teil der Rententionstoxine mit mittleren Molekulargewichten passieren, z. B. β2-Mikroglobulin (β2-M). Während bei der Low-Flux-Dialyse die Blutwäsche hauptsächlich auf Basis von diffusiven Prozessen stattfindet, profitiert die High-Flux-Dialyse neben den großen Porengrößen besonders von konvektiven (Filtratschleppe - solvent-drag) Prozessen.

Die Wand moderner vollsynthetischer Kapillarmembranen ist asymmetrisch aufgebaut. Kapillarmembranen aus Polysulfon zeigen auf der Innenseite eine dünne, etwa einen Mikrometer dicke Membranstruktur, die von einer ca. 40 µm dicken stabilsierenden und nach außen zunehmend poröseren Stützstruktur mechanisch gefestigt wird. Diese stützende Schicht ist aufgrund ihrer Polymerzusammensetzung in der Lage, verschiedene Moleküle bis hin zu Endotoxinen, Bakterien und Viren zu absorbieren.

In der WO 2013/106109 A1 ist ein Hämofilter zur Behandlung von Herzversagen offenbart. Dabei ist der Blutkreislauf außen um Kapillarmembranen herumgeführt. Aus dem Blut lagern sich Leukozyten an der Membranwand ab.

In der US 6,309,543 B1 ist ein Adapter offenbart. Der Adapter dient dazu, einen Dialysator zwischen verschiedenen Behandlungen zu desinfizieren.

In der EP 2 540 327 A1 werden verschiedene Anschlüsse für einen Dialysator beschrieben.

Im Artikel "Effects of Bleach Reprocessing on Hemodialysis Membranes" (Wolff, Susanne H., Zydney, Andrew L., "Effects of Bleach Reprocessing on Hemodialysis Membranes". The Pennsylvania State University, Dept. of Chemical Engineering, University Park, 2003, Vol. 752, Materials Research Society.) wird eine Hämodialyse Membran beschrieben, die durch Bleichen eine größere Porengröße erreicht.

Herkömmliche Dialysegeräte filtern nachteiligerweise und wegen deren entsprechender Größe keine Viren und Bakterien aus dem Blut heraus.

Es ist Aufgabe der Erfindung, ein Dialysegerät zur Verfügung zu stellen, das den oben genannten Nachteil vermeidet.

Die Erfindung ist ein Dialysegerät nach Anspruch 1 und macht von der Idee Gebrauch, die herkömmliche Ultrafitrationsrichtung entlang der Kapillarmembranen des Dialysators umzukehren. Dazu wird erfindungsgemäß der
Blutkreislauf entlang der Außenfläche der Kapillarmembran geführt und der Dialysatkreislauf entlang der Innenflächen der Kapillarmembranen geführt. Vorteilhafterweise strömt das Blut somit entlang der mit größeren Poren versehenen Außenfläche. In den größeren Poren können sich im Verlauf einer Ultrafiltration von außen nach innen Viren, Bakterien und Endotoxine verfangen und so durch Adsorption oder Größenausschluss dem Blut des Patienten entzogen werden. Der eigentliche Dialysevorgang, der über die Innenfläche mit geringer Porengröße stattfindet, erfolgt im Prinzip in herkömmlicher Weise.

Günstigerweise beträgt eine durchschnittliche Porengröße an einer Außenfläche der Kapillarmembran zwischen 1 - 4 µm, und günstigerweise beträgt eine durchschnittliche Porengröße einer Innenfläche einer Kapillarmembran weniger als 5 nm. Bakterien und Viren haben eine Größe zwischen 40 nm und einigen wenigen µm und gelangen somit in das Innere der Kapillarmembranwand über die großen Poren der Außenfläche und verfangen sich dort. Sie verbleiben also nicht wie bei der herkömmlichen Dialyse im Blutkreislauf, sondern werden aus diesem absorbiert.

Günstigerweise weisen die Kapillarmembranen in einem Querschnitt senkrecht zu einer Längsrichtung eine kreisförmige Innenfläche und eine kreisförmige Außenfläche auf, vorzugsweise sind die Kapillarmembranen entlang ihrer gesamten Längsausdehnung röhrenförmig ausgebildet und entlang ihrer gesamten Längsausdehnung im Querschnitt sowohl in der Innen- als auch in der Außenfläche kreisförmig ausgebildet. Die Kapillarmembranen sind leicht herstellbar.

Die Kapillarmembranen sind Teil eines Dialysators, der außen von einem Gehäuse gebildet ist. In dem Gehäuse, das in Längsrichtung röhrenförmig ausgeformt ist, ist das Bündel an Kapillarmembranen angeordnet. Die Kapillarmembranen sind vorzugsweise parallel zueinander angeordnet und an ihren offenen Enden in eine Halterung eingegossen, wodurch zwischen der Außenfläche der Kapillarmembranen und der Innenwandung des Gehäuses ein Blutkompartment mitausgebildet wird, das Teil des Blutkreislaufes ist, während Lumen der Kapillaren einen Abschnitt des Dialysatkreislaufes ausbilden und ein Dialysatkompartment bilden. Dialysatkompartment und Blutkompartment sind voneinander durch die semipermeablen Kapillarmembranen getrennt. Durch die Kapillarmembranen findet der eigentliche Ultrafiltrationsvorgang statt.

Vorzugsweise ist in dem sich in Längsrichtung erstreckenden Gehäuse das Bündel Kapillarmembrane in Längsrichtung verlaufend angeordnet, und das Blutkompartment weist zwei zweite Mutterkonnektortypen und das Dialysatkompartment zwei zweite Vaterkonnektortypen auf, die jeweils an sich gegenüberliegenden Enden in Längsrichtung des jeweiligen Kompartments angeordnet sind. Die zwei Mutterkonnektortypen wirken mit jeweils einem Blutkreislaufkonnektor zusammen, der jeweils an einem Ende eines Blutkreislaufschlauches angeordnet ist.

Erfindungsgemäß sind am Gehäuse des Dialysators zwei zweite Vaterkonnektoren angeordnet, und auf die zwei zweiten Vaterkonnektoren ist jeweils ein zweiter Adaptertyp aufgesetzt, der jeweils einen nach dem Aufsetzen freien zweiten Mutterkonnektor aufweist, der zum Anschluss an einen Blutkreislaufadapter bestimmt ist, und am Gehäuse des Dialysators sind zwei zweite Mutterkonnektoren angeordnet, und auf die zwei zweiten Mutterkonnektoren ist jeweils ein erster Adaptertyp aufgesetzt, der jeweils einen freien zweiten Vaterkonnketortyp aufweist, der zum Anschluss an einen Dialysatkreislauf bestimmt ist.

Durch den ersten und den zweiten Adaptertyp können die Blutkreislaufadapter und die Dialysatkreislaufadapter der herkömmlichen Dialysiermaschinen beibehalten werden, und gleichzeitig werden Blutkompartment und Dialysatkompartment miteinander vertauscht, um die erfindungswesesentliche Umkehrung der Ultrafiltrationsrichtung zu erzielen.

Die Erfindung wird anhand eines Ausführungsbeispiels in sieben Figuren beispielhaft beschrieben, dabei zeigen:
- Fig. 1: an einen Patienten angeschlossenen erfindungsgemäßen Dialysator,
- Fig. 2: perspektivische Ansicht einer Kapillarmembran,
- Fig. 3: Ausschnitt der Kapillarmembranwand mit asymmetrisch verteilten Poren,
- Fig. 4: Ansicht einer Innenfläche der Kapillarmembran in Fig. 2,

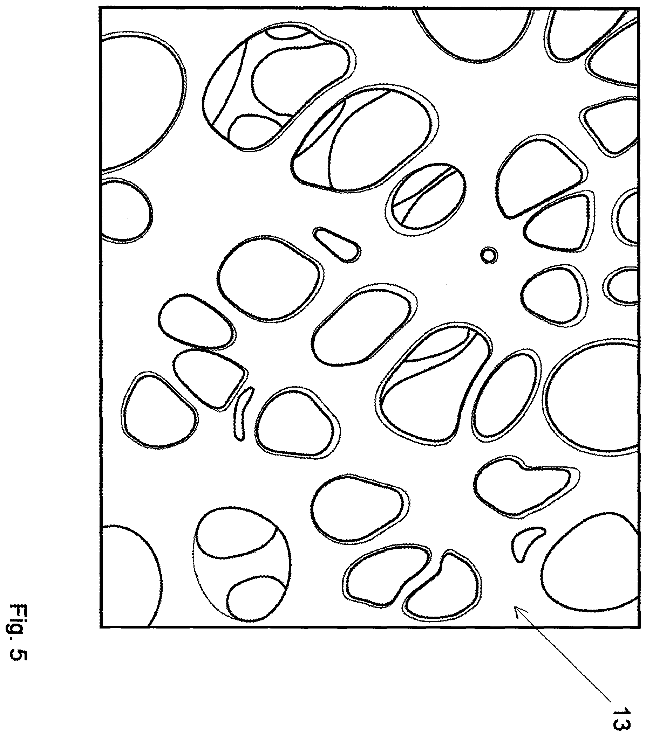

- Fig. 5: Ansicht einer Außenfläche der Kapillarmembran im gleichen Maßstab wie Fig. 4 und
- Fig. 6: einen Adapter eines ersten Typs und
- Fig. 7: einen Adapter eines zweiten Typs.

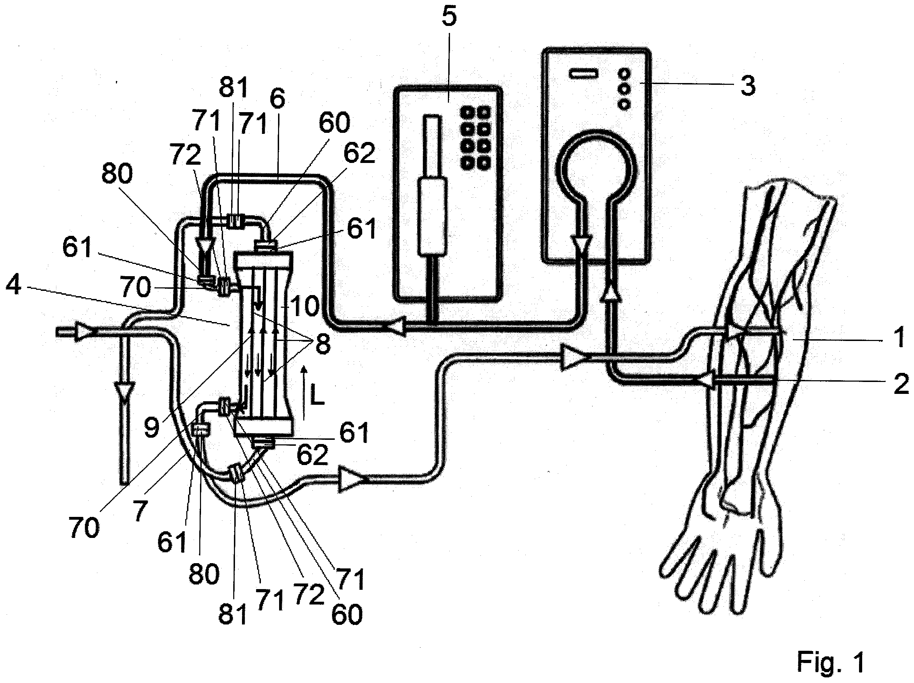

Fig. 1 zeigt schematisch den prinzipiellen Aufbau eines Dialysegerätes zur Durchführung einer Hämodialyse an einem Patienten. Dabei wird Blut 10 des Patienten extrakorporal gewaschen. Fig. 1 zeigt einen Unterarm 1 des Patienten. Das Blut 10 wird über einen Zugang 2 am Unterarm 1 des Patienten mittels eines Gefäßzugangs entnommen und mittels einer Blutpumpe 3 über den Zugang 2 gefördert und einem Dialysator 4 zugeführt. Dem dem Patienten entnommenen Blut 10 wird in einer Zuführeinrichtung 5 ein Gerinnungshemmer zusätzlich zugeführt, und das mit dem Gerinnungshemmer angereicherte Blut 10 wird in den Dialysator 4 gepumpt und im Dialysator 4 gewaschen. Der Dialysator 4 dient als die eigentliche "künstliche Niere", die Abfallprodukte aus dem durchfließenden Blut 10 des Patienten wäscht sowie dem durchfließenden Blut 10 Wasser entzieht.

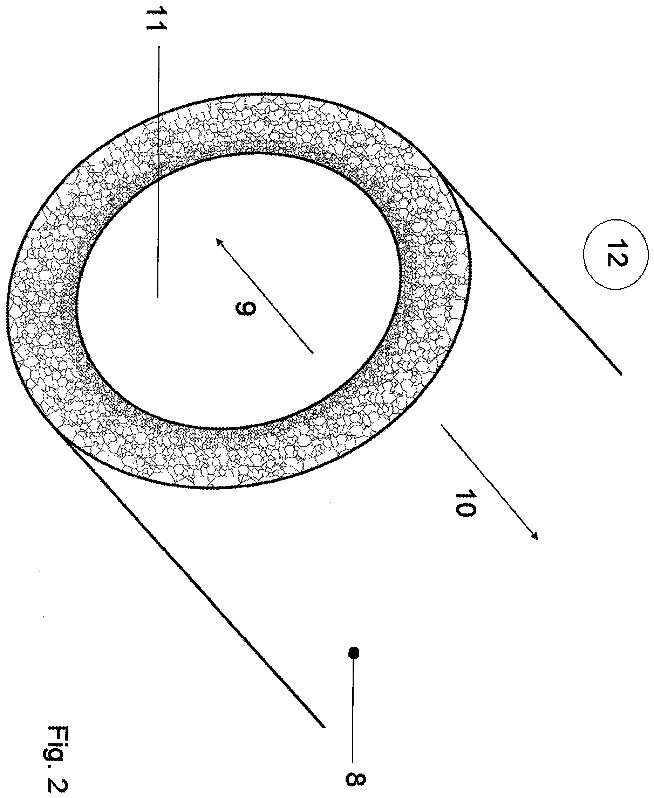

Der Dialysator 4 umfasst einen Abschnitt eines extrakorporalen Blutkreislaufs 6 und einen Abschnitt eines davon separierten Dialysatkreislaufs 7. Dem Dialysator wird durch den Blutkreislauf 6 Blut 10 und durch den Dialysatkreislauf 7 Dialysierflüssigkeit, auch Dialysat genannt, zugeführt. Der Abschnitt des Blutkreislaufs 6 und der Abschnitt des Dialysatkreislaufs 7 sind im Dialysator 4 gegenläufig und durch semipermeable Kapillarmembranen 8 voneinander getrennt. Die semipermeablen Kapillarmembranen 8 sind in Fig. 2 in einer perspektivischen Ansicht dargestellt. In Fig. 1 sind die Kapillarmembranen 8 durch drei durchgehende mit einer Fließrichtung gekennzeichnete Striche dargestellt. In dem Dialysator sind ca 10.000 Kapillarmembarnen nebeneinander, vorwiegend parallel zueinander angeordnet. Jede der Kapillarmembranen 8 wird vom Blut 10 des Abschnitts des Blutkreislaufs 6 umspült.

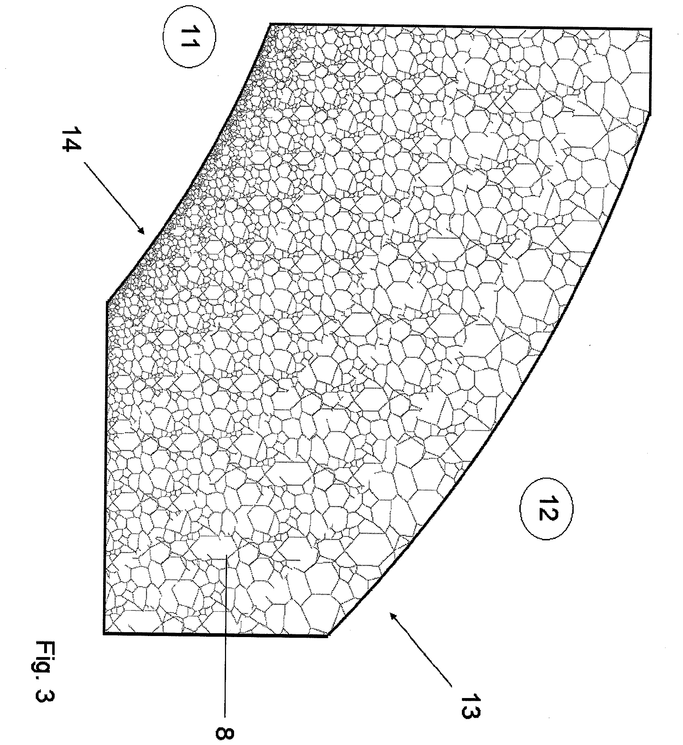

Der Dialysator 4 besteht gemäß Fig. 1 im Wesentlichen aus der Vielzahl der parallel nebeneinander in einer Längsrichtung L angeordneter Kapillarmembranen 8. Unter Kapillarmembranen 8 sind im Durchmesser kleine haarfeine Röhrchen zu verstehen, die einen Innendurchmesser von zwischen 150 µm und 240 µm und einen Außendurchmesser zwischen 200 µm und größer als 300 µm aufweisen. Die Kapillarmembranen 8 sind parallel nebeneinander, vorzugsweise ohne direkten Kontakt zueinander in dem Dialysator 4 angeordnet. Durch jeweils ein Lumen 11 jeder der Kapillarmembranen 8, d. h. durch die jeweils freie Innenröhre der Kapillarmembranen 8 fließt ein Dialysat 9 des Dialysatkreislaufes 7 in Längsrichtung L jeder der Kapillarmembranen 8. In einem die Kapillarmembran 8 umgebenden Außenraum 12, der in den Fign. 2, 3 dargestellt ist, jeder der Kapillarmembranen 8 fließt in die entgegengesetzte Richtung, also entgegen der Längsrichtung L, das Blut 10 im Blutkreislauf 6 an den Kapillarmembranen 8 außen vorbei. Die Kapillarmembranen 8 sind alle jeweils semipermeabel ausgebildet.

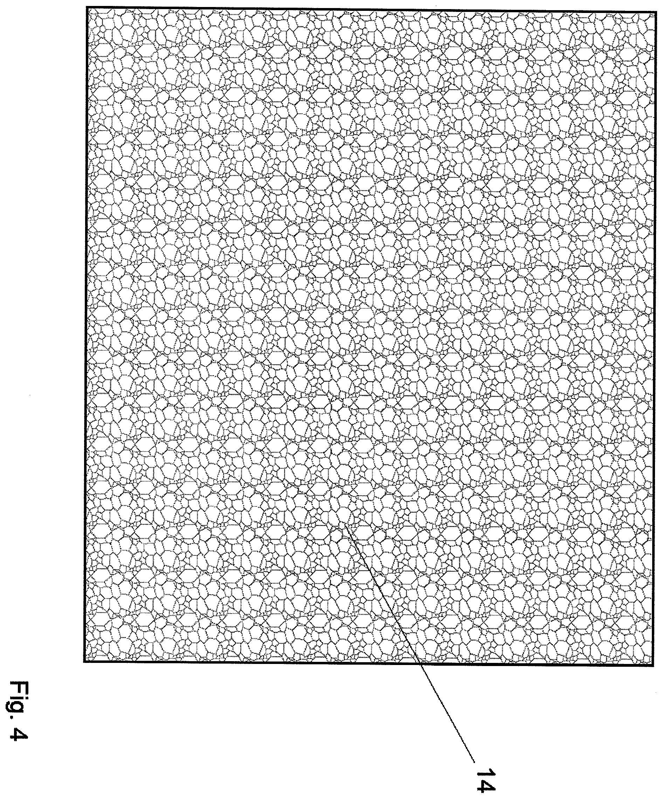

Die Fig. 3 zeigt einen Ausschnitt der Wand der Kapillarmembran 8 in Fig. 2. Die Wand der Kapillarmembran 8 besteht vorzugsweise aus voll synthetischen Polymeren und ist in radialer Richtung asymmetrisch aufgebaut. In Längsrichtung L ist die Kapillarmembran 8 hingegen im Wesentlichen translationsinvariant ausgebildet. Die Kapillarmembranen 8 weisen eine Außenfläche 13 und eine Innenfläche 14 auf, wobei die Innenfläche 14 eine sehr feine Membranstruktur, d. h. eine geringe Membrandicke von etwa 1 µm und einer Porengröße von <5nm im Durchschnitt aufweist, während die Kapillarmembranwand 8 in Richtung Außenoberfläche 13 eine zunehmend größere Porengröße aufweist und die Porengröße hier vorzugsweise 1 - 4 µm beträgt. Die Innenfläche 14 der Kapillarmembran 8 wird auch durch die zunehmend porösere Stützstruktur mechanisch gefestigt. Die unterschiedlichen Porengrößen der Außenfläche 13 und Innenfläche 14 werden durch einen Vergleich der im gleichen Maßstab dargestellten Figuren 3 und 4 deutlich.

Im Gegensatz zu herkömmlichen Dialysegeräten sind erfindungsgemäß der Blutkreislauf 6 und der Dialysatkreislauf 7 vertauscht. Bei einer herkömmlichen Dialysebehandlung werden bekanntlich das Blut 10 durch das Lumen 11 der Kapillarmembranen 8 und das Dialysat 9 im Gegenstrom außen um die Kapillarmembranen 8 geleitet. Erfindungsgemäß wird genau umgekehrt verfahren, indem das Dialysat 9 durch das Lumen 11 der Kapilllarmembranen 8 fließt und das Blut 10 die Kapillarmembran 8 von außen umströmt. Dabei wird auch die Richtung der Ultrafiltration umgekehrt und findet von außen in Richtung des Lumens 11 der Kapillarmembran 8 statt. Als Konsequenz der umgekehrten Richtung der Ultrafiltration steht eine im Vergleich zur herkömmlichen Dialyse größere Absorptionsfläche von ca. 1.500 m² zur Verfügung. Dazu wird die durch die Poren gebildete sehr große Innenfläche der Kapillarmembran 8 schätzungsweise bestimmt. Die große Absorptionsfläche in der Wand der Kapillarmembran 8 kann für das Entfernen von großmolekularen Toxinen, Bakterien oder Viren genutzt werden, die während der Ultrafiltration gleichsam in den Poren der Kapillarmembran 8 steckenbleiben, da die Porosität von außen nach innen feiner wird. Gleichzeitig findet nach wie vor eine Dialyse in den Kapillarmembranen 8 durch Diffusion und Konvektion, je nach Dialysatortyp und Verfahren, statt.

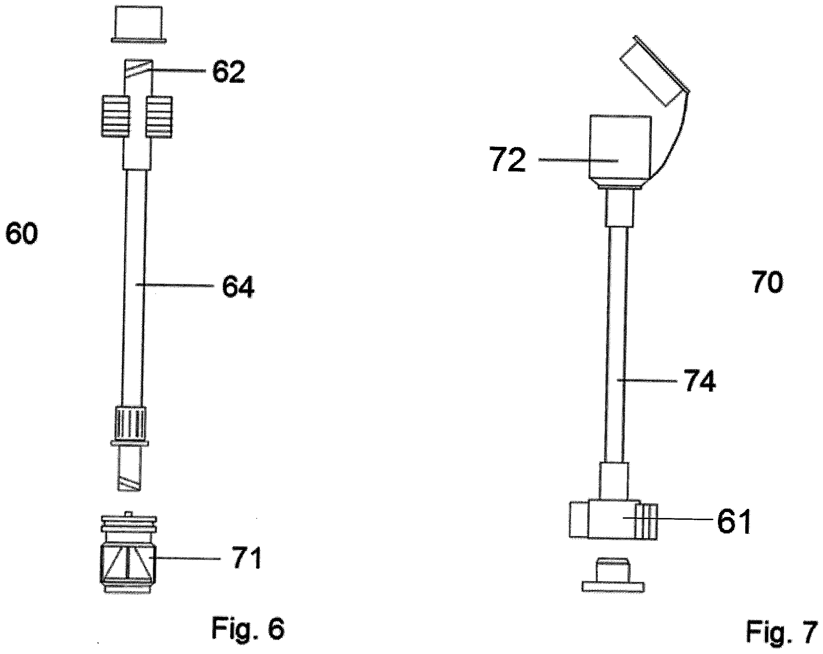

Vorteilhafterweise kann ein herkömmlicher Dialysator 4 als Bauteil des erfindungsgemäßen Dialysegrätes Verwendung finden. Dazu wird der Dialysator 4 durch zwei erste und zwei zweite Adaptertypen 60, 70 in den Fig. 6 bzw. 7 erweitert. Der Dialysator 4 weist zwei zweite Mutterkonnektortypen 61 auf, die in Fig. 1 an den Stirnseiten des Dialysators 4 vorgesehen sind und in flüssigkeitsleitender Verbindung mit den Lumen 11 der Kapillarmambranen 8 stehen. Der Dialysator 4 weist an der zylindrischen Außenwandung zwei zweite Vaterkonnektortypen 71 auf.

Der erste Adaptertyp 60 weist einen ersten Vaterkonnektortyp 62 und einen zweiten Vaterkonnektortyp 71 auf. Erster Vaterkonnektortyp 62 und zweiter Vaterkonnektortyp 71 sind an verschiedenen Enden eines ersten Schlauches 64 angeordnet und liegen sich gegenüber.

Der zweite Adaptertyp 70 weist einen ersten Mutterkonnektortyp 72 und einen zweiten Mutterkonnektortyp 61 auf. Erster Mutterkonnektortyp 72 und zweiter Mutterkonnektortyp 61 sind an verschiedenen Enden eines zweiten Schlauches 74 sich gegenüberliegend angeordnet.

Die zweiten Vaterkonnektortypen 71, die an der Außenwandung des Dialysators 4 angeordnet sind, und die zweiten Vaterkonnektortypen 71 am ersten Adapterertyp 60 sind nicht notwendigerweise baugleich, sondern lediglich funktionsgleich, und zwar in dem Sinne, dass sie mit einem ersten Mutterkonnektortyp 72 eine flüssigkeitsdichte Verbindung ausbilden. Entsprechendes gilt für den zweiten Mutterkonnektortyp 61, der eine flüssigkeitsdichte Verbindung mit dem ersten Vaterkonnektortyp 62 ausbilden muss.

An die zweiten Mutterkonnektortypen 61 des Dialysators 4 ist jeweils ein erster Adaptertyp 60 mit seinem ersten Vaterkonnektortyp 62 aufgesetzt. Bei dem zweiten Mutterkonnektortyp 61 handelt es sich um ein Innengewinde, beim ersten Vaterkonnektortyp 62 um ein Außengewinde.

Auf den zweiten Vaterkonnektortyp 71 ist jeweils ein zweiter Adaptertyp 70 mit seinem ersten Mutterkonnektortyp 72 aufgesetzt. Dabei liegt eine Steckverbindung vor.

Die beiden zweiten Mutterkonnektortypen 61, die vom zweiten Adaptertyp 70 abgehen, sind jeweils mit einem Blutkreislaufadapter 80 verbunden. Die beiden zweiten Vaterkonnektortypen 71 sind jeweils mit einem Dialysatkreislaufadapter 81 verbunden. Die beiden Adaptertypen 60, 70 vertauschen damit die Konnektortypen herkömmlicher Dialysatoren 4.

### Bezugszeichenliste

- L: Längsrichtung

- 1: Unterarm
- 2: Zugang
- 3: Blutpumpe
- 4: Dialysator

- 5: Zuführeinrichtung für Antikoagulation
- 6: Blutkreislauf
- 7: Dialysatkreislauf
- 8: Kapillarmembranen

- 9: Dialysat
- 10: Blut

- 11: Lumen
- 12: Außenraum
- 13: Außenfläche
- 14: Innenfläche

- 60: erster Adaptertyp
- 61: Mutterkonnektor zweiten Typs
- 62: Vaterkonnektor ersten Typs

- 64: erster Schlauch

- 70: zweiter Adaptertyp
- 71: Vaterkonnektor zweiten Typs
- 72: Mutterkonnektor ersten Typs
- 74: zweiter Schlauch

- 80: Blutkreislaufadapter
- 81: Dialysatkreislaufadapter

## Patentansprüche

1. Dialysegerät mit einem Dialysator (4) mit einem Bündel an Kapillarmembranen (8) mit jeweils einer Außenfläche (13) und einer Innenfläche (14) und mit einer im Bereich der Außenfläche (13) größeren durchschnittlichen Porengröße als im Bereich der Innenfläche (14), einem Blutkreislauf (6) und einem Dialysatkreislauf (7),
wobei der Blutkreislauf (6) entlang der Außenflächen (13) vorgesehen ist und der Dialysatkreislauf (7) entlang der Innenflächen (14) vorgesehen ist und die Kapillarmembranen (8) Lumen (11) umgeben, die Teil des Dialysatkompartments sind und ein die Kapillarmembranen (8) außen umgebender Raum (12) Teil des Blutkompartments ist und
die Kapillarmembran eine von der Kapillarinnenfläche (14) zur Kapillaraußenfläche (13) zunehmend größere Porengröße aufweist, **dadurch gekennzeichnet, dass** am Gehäuse zwei zweite Vaterkonnektortypen (71) angeordnet sind, die mit dem Blutkompartment verbunden sind, und außen auf die zwei zweiten Vaterkonnektortypen (71) jeweils ein Adapter zweiten Typs (70) gesetzt ist und am Gehäuse zwei zweite Mutterkonnektortypen (61) angeordnet sind, die mit dem Dialysatkompartment verbunden sind, und auf die zwei zweiten Mutterkonnektortypen (61) jeweils ein Adapter ersten Typs (60) gesetzt ist und
der Adapter ersten Typs (60) einen ersten Vaterkonnektortyp (62) und einen zweiten Vaterkonnektortyp (71) aufweist und
der Adapter zweiten Typs (70) einen ersten Mutterkonnektortyp (72) und einen zweiten Mutterkonnektortyp (61) aufweist.

2. Dialysegerät nach Anspruch 1,
**dadurch gekennzeichnet, dass** eine durchschnittliche Porengröße an der Kapillaraußenfläche (13) zwischen 1 - 4 µm beträgt.

3. Dialysegerät nach Anspruch 1 oder 2,
**dadurch gekennzeichnet, dass** eine durchschnittliche Porengröße an der Kapillarinnenfläche (14) weniger als 5 nm beträgt.

4. Dialysegerät nach Anspruch 1, 2 oder 3,
**dadurch gekennzeichnet, dass** die Kapillarmembranen (8) in einem Querschnitt eine kreisförmige Innenfläche (14) und eine kreisförmige Außenfläche (13) aufweisen.

5. Dialysegerät nach einem der vorstehenden Ansprüche,
**gekennzeichnet durch** ein sich in einer Längsrichtung (L) erstreckendes Gehäuse, in dem das Bündel Kapillarmembranen (8) in Längsrichtung (L) verlaufend angeordnet ist, und das Blutkompartment weist zwei zweite Mutterkonnektortypen (61) und das Dialysatkompartment zwei zweite Vaterkonnektortypen (71) auf.

## Claims

1. Dialysis apparatus comprising a dialyser (4) with a cluster of capillary membranes (8) each having an outer face (13) and an inner face (14) and a mean pore size which is larger in the region of the outer face (13) than in the region of the inner face (4), a blood circulation system (6) and a dialysate circulation system (7), whereby the blood circulation system (6) is provided along the outer faces (13) and the dialysate circulation system (7) is provided along the inner faces (14) and the capillary membranes (8) surround lumens (11) which are part of the dialysate compartment, and a chamber (12) surrounding the capillary membranes (8) on the outside is part of the blood compartment and the capillary membrane has a pore size which increases from the inner face (14) to the outer face (13) **characterized in that** two second male connector types (71) are arranged on the housing which are connected to the blood compartment, and an adapter of a second type (70) is placed on the outside on each of the two second male connector types (71), and two second female connector types (61) are arranged on the housing which are connected to the dialysate compartment, and an adapter of a first type (60) is placed on each of the two second female connector types (61) and the adapter of the first type (60) has a first male connector type (62) and a second male connector type (71) and the adapter of the second type (70) has a first female connector type (72) and a second female connector type (61).

2. Dialysis apparatus according to claim 1, **characterised in that** a mean pore size on the capillary outer face (13) is between 1 - 4 µm.

3. Dialysis apparatus according to claim 1 or 2, **characterised in that** a mean pore size on the capillary inner face (14) is less than 5 nm.

4. Dialysis apparatus according to claim 1, 2 or 3, **characterised in that** the capillary membranes (8) in a cross-section have a circular inner face (14) and a circular outer face (13).

5. Dialysis apparatus according to any of the preceding claims, **characterised by** a housing extending in the longitudinal direction (L) in which the cluster of capillary membranes (8) is arranged running in the longitudinal direction (L), and the blood compartment has two second female connector types (61) and the dialysate compartment has two second male connector types (71).

## Revendications

1. Appareil de dialyse avec un dialyseur (4) avec un faisceau de membranes capillaires (8) avec chacune une surface extérieure (13) et une surface intérieure (14) et avec une grandeur de pores moyenne plus grande dans la zone de la surface extérieure (13) que dans la zone de la surface intérieure (14), un circuit sanguin (6) et un circuit de dialysat (7), le circuit sanguin (6) étant prévu le long des surfaces extérieures (13) et le circuit de dialysat (7) étant prévu le long des surfaces intérieures (14) et les membranes capillaires (8) entourant des lumens (11) qui sont une partie du compartiment du dialysat et un espace (12) qui entoure les membranes capillaires (8) à l'extérieur étant une partie du compartiment du sang et la membrane capillaire présentant une grandeur de pores qui va croissante de la surface intérieure de la capillaire (14) vers la surface extérieure de la capillaire (13), **caractérisé en ce que** deux seconds types de connecteur père (71) qui sont reliés au compartiment du sang sont placés sur le boîtier et un adaptateur du second type (70) est mis à l'extérieur sur chacun des deux seconds types de connecteur père (71) et deux seconds types de connecteur mère (61) qui sont reliés au compartiment du dialysat sont placés sur le boîtier et un adaptateur du premier type (60) est mis sur chacun des deux seconds types de connecteur mère (61) et l'adaptateur de premier type (60) présente un premier type de connecteur père (62) et un second type de connecteur père (71) et l'adaptateur de second type (70) présente un premier type de connecteur mère (72) et un second type de connecteur mère (61).

2. Appareil de dialyse selon la revendication 1, **caractérisé en ce qu'**une grandeur de pores moyenne sur la surface extérieure de la capillaire (13) est comprise entre 1 et 4 µm.

3. Appareil de dialyse selon la revendication 1 ou 2, **caractérisé en ce qu'**une grandeur de pores moyenne sur la surface intérieure de la capillaire (14) est inférieure à 5 nm.

4. Appareil de dialyse selon la revendication 1, 2 ou 3, **caractérisé en ce que** les membranes capillaires (8) présentent dans une section transversale une surface intérieure (14) circulaire et une surface extérieure circulaire (13).

5. Appareil de dialyse selon l'une des revendications précédentes, **caractérisé par** un boîtier qui s'étend dans une direction longitudinale (L) dans lequel le faisceau de membranes capillaires (8) est placé dans la direction longitudinale (L) et le compartiment du sang présente deux seconds types de connecteur mère (61) et le compartiment du dialysat deux seconds types de connecteur père (71).
